# EUROPEAN PATENT APPLICATION

(11) **EP 2 301 589 A1**
(43) Date of publication of application: **30.03.2011**
(21) Application number: 09754546.1
(22) Date of filing: 07.05.2009
(51) Int. Cl.: A61L 9/14, A61L 9/16, A61L 9/22, B01D 46/42, F24F 1/00, F24F 1/02

(54) **AIR PURIFIER**

(30) Priority: 27.05.2008 JP 2008138650
(71) Applicant: Panasonic Electric Works Co., Ltd., Kadoma-shi, Osaka 571-8686 (JP)
(72) Inventor: ASANO, Yukiyasu, Kadoma-shi Osaka 571-8686 (JP); MIHARA, Fumio, Kadoma-shi Osaka 571-8686 (JP); URATANI, Yutaka, Kadoma-shi Osaka 571-8686 (JP)
(74) Representative: Appelt, Christian W.
(86) International application number: PCT/JP2009/058634
(87) International publication number: WO 2009/145038

(57) **Abstract**

The air purification apparatus includes a duct (10), a filter (20) for air purification, an electrostatically atomizing device (30), a fan (40), and a switching device (50). The duct (10) has an inlet (11) and an outlet (12). The filter (20) is placed inside of the duct (10). The electrostatically atomizing device (30) is configured to generate a mist of charged minute water particles by means of electrostatically atomizing phenomenon and discharge the same into the duct (10). The fan (40) is configured to send air from the inlet (11) to the outlet (12). The switching device (50) is configured to switch one of a first mode and a second mode. The air purification apparatus is configured to send a mist of charged minute water particles from the electrostatically atomizing device (30) to the outlet (12) not through the filter (20) in said first mode. The air purification apparatus is configured to send a mist of charged minute water particles from the electrostatically atomizing device (30) to the filter (20) in the second mode.

## Description

### Technical Field

The present invention is directed to air purification apparatuses, and more particularly to an air purification apparatus including an electrostatically atomizing device.

### Background Art

As disclosed in Japanese patent laid-open publication No. 2004-85185 (hereinafter referred to as "document 1"), and Japanese patent laid-open publication No. 2005-180865 (hereinafter referred to as "document 2"), there has been proposed an air purification apparatus provided with an electrostatically atomizing device.

In the air purification apparatuses respectively disclosed in the documents 1 and 2, a filter is placed inside of an air duct.

Notably, concerning the air purification apparatus disclosed in the document 1, the electrostatically atomizing device is placed downstream of the filter. Therefore, a mist of charged minute water particles generated by the electrostatically atomizing device is discharged into an external space not through the filter. The air purification apparatus disclosed in the document 1 discharges a mist of charged minute water particles into the external space in order to purify air in the external space by use of a mist of charged minute water particles.

In the air purification apparatus disclosed in the document 2, the electrostatically atomizing device is placed upstream of the filter. Therefore, a mist of charged minute water particles generated by the electrostatically atomizing device comes into contact with the filter. The air purification apparatus disclosed in the document 2 makes a mist of charged minute water particles into contact with the filter in order to regenerate the filter by use of a mist of charged minute water particles.

As apparent from the above configurations, the air purification apparatus of the document 1 does not utilize the electrostatically atomizing device to regenerate the filter, and the air purification apparatus of the document 2 does not utilize the electrostatically atomizing device to purify air in the external space.

In order to make both air purification and filter regeneration, the electrostatically atomizing devices can be placed both downstream and upstream of the filter.

However, with this situation, at least two electrostatically atomizing devices are required. As a result, the air purification apparatus is likely to be upsized and its cost is likely to become high.

### Disclosure of Invention

In view of the above insufficiency, the present invention has been aimed to propose an air purification apparatus capable of making both air purification and filter regeneration by use of a single electrostatically atomizing device.

The air purification apparatus in accordance with the present invention includes a duct, a filter designed to purify air, an electrostatically atomizing device, a fan, and a switching means. The duct has an inlet and an outlet. The filter is placed inside of the duct. The electrostatically atomizing device is configured to generate a mist of charged minute water particles by means of electrostatically atomizing phenomenon and discharge the same into the duct. The fan is configured to send air from the inlet to the outlet. The switching means is configured to switch one of a first mode and a second mode. The air purification apparatus is configured to send a mist of charged minute water particles from the electrostatically atomizing device to the outlet without passing through the filter in the first mode. The air purification apparatus is configured to send a mist of charged minute water particles from the electrostatically atomizing device to the filter in the second mode.

According to the air purification apparatus in accordance with the present invention, it is possible to purify air in the first mode, and to regenerate the filter in the second mode. Further, the first mode and the second mode can be switched by use of the switching means. Consequently, the air purification apparatus in accordance with the present invention is capable of making both air purification and filter regeneration by use of the single electrostatically atomizing device. Therefore, it is possible to downsize the air purification apparatus, and to reduce a production cost. In addition, in the first mode, a mist of charged minute water particles is discharged out from the duct not through the filter. Thus, it is possible to prevent occurrence of a decrease of an amount of a mist of charged minute water particles discharged out from the duct caused by the filter. As a result, it is possible to provide an enough amount of a mist of charged minute water particles discharged out from the duct. Moreover, since the filter can be regenerated in the second mode, it is possible to reduce replacement frequency of filter. Thus, maintenance need not be made for a long time.

In a preferred embodiment, the electrostatically atomizing device is configured to discharge a mist of charged minute water particles into a space formed inside of the duct between the filter and the outlet. The switching means includes a changing means and a controlling means configured to control the changing means. The changing means is configured to change a discharge direction where the electrostatically atomizing device discharges a mist of charged minute water particles. The controlling means is configured to direct the discharge direction toward the outlet when the first mode is selected. The controlling means is configured to direct the discharge direction toward the filter when the second mode is selected.

With the preferred embodiment, it is possible to easily and successfully select one of the first mode and the second mode.

In a preferred embodiment, the electrostatically atomizing device is configured to discharge a mist of charged minute water particles into a space formed inside of the duct between the filter and the outlet. The fan is configured to operate in an air supply mode of sending air from the inlet to the outlet, and an exhaust mode of sending air from the outlet to the inlet. The switching means includes a controlling means configured to control the fan. The controlling means is configured to control the fan to operate in the air supply mode when the first mode is selected, and to control the fan to operate in the exhaust mode when the second mode is selected.

With the preferred embodiment, it is possible to easily and successfully select one of the first mode and the second mode.

In a preferred embodiment, the electrostatically atomizing device is placed inside of the duct. The duct includes, between the outlet and the electrostatically atomizing device, a first air passage devoid of the filter and a second air passage provided with the filter. The switching means includes a shutter configured to block the first air passage and the second air passage selectively. The switching means includes a controlling means configured to control the shutter. The controlling means is configured to control the shutter to block the second air passage when the first mode is selected. The controlling means is configured to control the shutter to block the first air passage when the second mode is selected.

With the preferred embodiment, it is possible to easily and successfully select one of the first mode and the second mode.

In a preferred embodiment, the electrostatically atomizing device is configured such that an amount of a mist of charged minute water particles generated per unit time is greater in the second mode than in the first mode.

With the preferred embodiment, it is possible to shorten time necessitated for regenerating the filter.

### Brief Description of Drawings

FIG. 1A is a schematic explanatory view illustrating an air purification apparatus of a first embodiment operating in a first mode,
FIG. 1B is a schematic explanatory view illustrating the air purification apparatus of the first embodiment operating in a second mode,
FIG. 2 is a schematic explanatory view illustrating an electrostatically atomizing device of the above air purification apparatus,
FIG. 3 is an explanatory view illustrating a modification of the above air purification apparatus,
FIG. 4A is a schematic explanatory view illustrating an air purification apparatus of a second embodiment operating in a first mode,
FIG, 4B is a schematic explanatory view illustrating the air purification apparatus of the second embodiment operating in a second mode,
FIG. 5A is a schematic explanatory view illustrating an air purification apparatus of a third embodiment operating in a first mode,
FIG. 5B is a schematic explanatory view illustrating the air purification apparatus of the third embodiment operating in a second mode, and
FIG. 6 is an explanatory view illustrating a modification of the above air purification apparatus.

### Best Mode for Carrying Out the Invention

### (first embodiment)

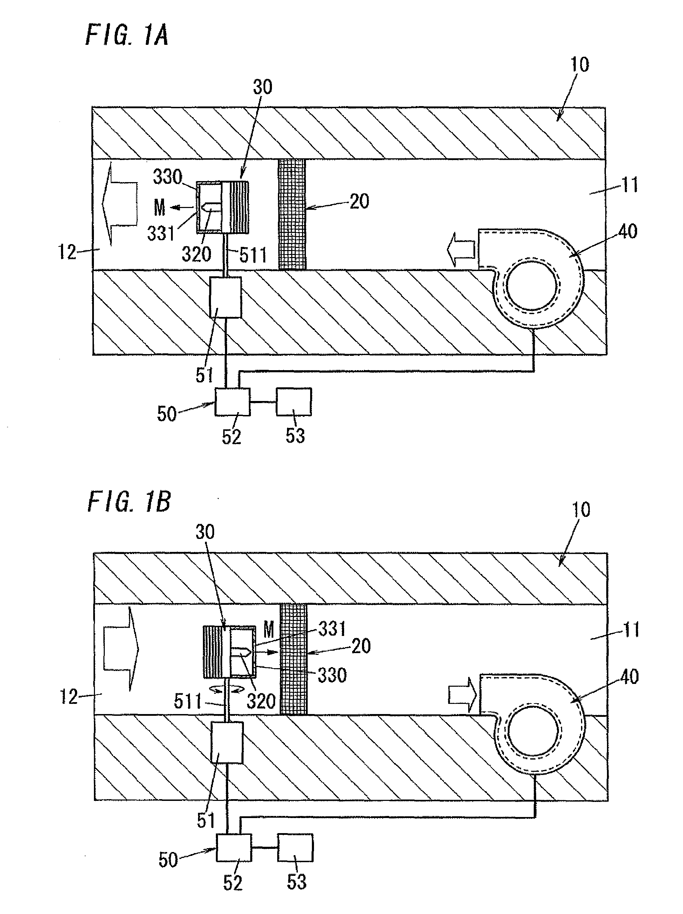

As shown in FIGS. 1A and 1B, an air purification apparatus of the present embodiment includes a duct **10,** a filter **20** for air purification, an electrostatically atomizing device **30,** a fan **40,** and a switching device (switching means) **50.**

The duct **10** is mounted on a housing which is a casing of the air purification apparatus. The duct **10** is shaped into a cylindrical shape, for example. A first axial opening (right opening, in FIGS. 1A and 1B) of the duct **10** is defined as an inlet **11.** The inlet **11** is used for introducing air into the duct **10** from the outside. A second axial opening (left opening, in FIGS. 1A and 1B) of the duct **10** is defined as an outset **12.** The outlet **12** is used for discharging air to the outside from the inside of the duct **10.**

The filter **20** is placed inside of the duct **10.** For example, the filter **20** is a deodorization filter configured to remove offensive odor substances from air utilizing an activated carbon.

The fan **40** is placed close to the inlet **11** within the duct **10.** The fan **40** is configured to operate in two working modes, one being an air supply mode and the other being an exhaust mode. The fan **40** sends air from the inlet **11** to the outlet **12** in the air supply mode. The fan **40** sends air from the outlet **12** to the inlet **11** in the exhaust mode. In the present embodiment, based on a situation where the fan **40** operates in the air supply mode, a side of the inlet **11** of the duct **10** is defined as an upstream side, and a side of the outlet **12** of the duct **10** is defined as a downstream side.

The electrostatically atomizing device **30** is configured to generate a mist of charged minute water particles by means of electrostatically atomizing phenomenon and discharge the same into the duct **10.** In the present embodiment, the electrostatically atomizing device **30** is placed between the filter **20** and the outlet **12** in the duct **10.** Consequently, the electrostatically atomizing device **30** discharges a mist of charged minute water particles into a space formed inside of the duct **10** between the filter **20** and the outlet **12.**

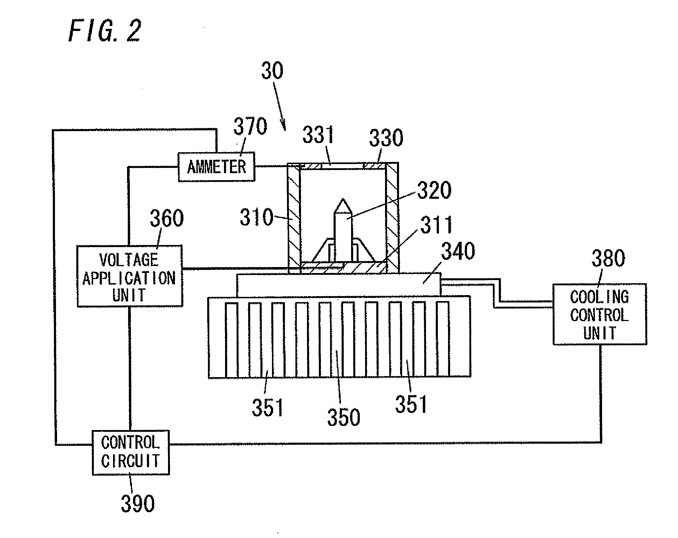

As shown in FIG. 2, the electrostatically atomizing device **30** includes a housing **310.**

The housing **310** is made of a dielectric material and is shaped into a cylindrical shape. The first axial opening (lower opening shown in FIG. 2) of the housing **310** is covered with a cooling plate **311.** The housing **310** houses a discharge electrode **320.**

The discharge electrode **320** is shaped into a bar shape having a sharp tip. The discharge electrode **320** has its base end thermally coupled to the cooling plate **311**.

There is an opposed electrode **330** which is placed inside of a second opening (upper opening shown in FIG. 2) of the housing **310.**

The opposed electrode **330** is shaped into a plate shape having enough dimensions to cover the second opening of the housing **310**. The opposed electrode **330** is provided with a spray hole **331** in its center.

The tip of the discharge electrode **320** is visible from the outside of the housing **310** via the spray hole **331.**

A peltier unit **340** having a peltier element (not shown) is attached to the first opening side of the housing **310.**

The peltier unit **340** is used for cooling the discharge electrode **320.** Therefore, the peltier unit **340** has its cooling side thermally coupled to the discharge electrode **320** through the cooling plate **311**. The peltier unit **340** has its heat dissipation side attached to a heat dissipation unit **350** having plural fins **351.**

The electrostatically atomizing device **30** includes, as circuit components, a voltage application unit **360,** an ammeter **370,** a cooling control unit **380,** and a control circuit **390.**

The voltage application unit **360** is configured to apply a voltage (DC voltage) between the discharge electrode **320** and the opposed electrode **330.** For example, the voltage application unit **360** is an AC/DC converter which is configured to apply a voltage between the discharge electrode **320** and the opposed electrode **330** by use of power received from a commercial AC source.

The ammeter **370** is configured to measure a current flowing between the discharge electrode **320** and the opposed electrode **330.**

The cooling control unit **380** is configured to energize the peltier element of the peltier unit **340**. The cooling control unit **380** has a function of adjusting a voltage applied to the peltier element.

The control circuit **390** is constructed by use of a microcomputer, for example.

The control circuit **390** is configured to control the cooling control unit **380** to cool the discharge electrode **320** below a dew point of circumambient air. When the discharge electrode **320** is cooled below the aforementioned dew point, moisture in the circumambient air is condensed on the surface of the discharge electrode **320.** Consequently, dew is produced on the surface of the discharge electrode **320.** That is, the electrostatically atomizing device **30** of the present embodiment is configured to supply water (dew condensation water) to the discharge electrode **320** utilizing dew condensation (surface condensation).

Especially, the control circuit **390** of the present embodiment adjusts the temperature of the discharge electrode **320** to either a first configuration temperature or a second configuration temperature. Both the first configuration temperature and the second configuration temperature are selected to be not greater than the above dew point. The second configuration temperature is lower than the first configuration temperature. Therefore, the discharge electrode **320** develops the dew on its surface in a greater amount when it is at second preset temperature than at the first preset temperature.

The control unit **390** is configured to control the voltage application unit **360** with reference to a detection result of the ammeter **370** in order to apply a predetermined voltage (e.g., 500V) between the discharge electrode **320** and the opposed electrode **330.** Therefore, water held by the discharge electrode is atomized, and then a mist of charged minute water particles is generated. A mist of charged minute water particles is discharged out of the housing **310** via the spray hole **331** of the opposed electrode **330**. A principle of the electrostatically atomizing is well known, and no explanation thereof is deemed necessary.

The air purification apparatus of the present embodiment is configured to operate in two operation modes, one being a first mode and the other being a second mode. In the first mode, the air purification apparatus sends a mist of charged minute water particles from the electrostatically atomizing device to the outlet **12** not through the filter **20** (see FIG. 1A). In the second mode, the air purification apparatus sends a mist of charged minute water particles from the electrostatically atomizing device to the filter **20** (see FIG. 1B). Besides, arrows respectively shown in FIG. 1A and FIG. 1B indicate a direction of air.

The switching device **50** is configured to switch one of the first mode and the second mode.

The switching device **50** includes an electric motor **51,** a control unit **52,** and a manipulation unit **53.**

The electric motor **51** is a stepping motor, for example. The electric motor **51** has a stator (not shown) fixed to the duct **10.** The electric motor **51** includes a rotation axis **511** configured to rotate with a rotor (not shown). The rotation axis **511** has its tip fixed to the electrostatically atomizing device **30.** A discharge direction **M** in which the electrostatically atomizing device **30** discharges a mist of charged minute water particles is perpendicular to an axial direction of the rotation axis **511.** Therefore, with a rotation of the rotation axis **511**, the discharge direction **M** is changed. In the present embodiment, the electric motor **51** is defined as a changing means configured to change the discharge direction **M.**

The manipulation unit **53** is used for making the switching one of the first mode and the second mode by manual operation, for example. The manipulation unit **53** is constructed by use of a switch or button, for example.

The control unit **52** is configured to control the electric motor **51,** the fan **40,** and the control circuit **390** in accordance with the operation mode of the air purification apparatus. The control unit **52** is configured to switch one of the first mode and the second mode corresponding to manual operation of the manipulation unit **53.** The control unit **52** is constructed by use of a microcomputer, for example.

When the first mode is selected, the control unit **52** rotates the rotation axis **511** of the electric motor **51** to direct the discharge direction **M** toward the outlet **12** (downstream side). In addition, the control unit **52** controls the fan **40** to operate in the air supply mode. Further, the control unit **52** controls the control circuit **390** to adjust the temperature of the discharge electrode **320** to the first configuration temperature.

Therefore, in the first mode, a mist of charged minute water particles is discharged from the electrostatically atomizing device **30** towards the outlet **12.** Further, since the fan **40** sends air towards the downstream side, a mist of charged minute water particles is moved towards the outlet **12** as being carried on an air flow.

As seen from the above, the first mode is defined as a purification mode (external space purification mode) of discharging out a mist of charged minute water particles via the outlet **12** without passing through the filter **20.** When discharged into the external space, the mist of charged minute water particles remains floating in the air for a long time, and spread through every corner of the space. Thus, radical species contained in a mist of charged minute water particles removes offensive odor substances from the external space.

When the second mode is selected, the control unit **52** rotates the rotation axis **511** of the electric motor **51** to direct the discharge direction **M** toward the filter **20** (upstream side). In addition, the control unit **52** controls the fan **40** to operate in the exhaust mode. Further, the control unit **52** controls the control circuit **390** to adjust the temperature of the discharge electrode **320** to the second configuration temperature. Besides, in the second mode, the fan **40** may be deactivated instead of operating in the exhaust mode (it is sufficient that the fan **40** does not operate in the air supply mode).

Therefore, in the second mode, a mist of charged minute water particles is discharged from the electrostatically atomizing device **30** towards the filter **20.** Further, since the fan **40** sends air towards the upstream side, a mist of charged minute water particles is moved towards the filter **20** as being carried on an air flow,

As seen from the above, the second mode is defined as a regeneration mode (filter regeneration mode) of spraying the mist of charged minute water particles into the filter **20** for regeneration thereof. When contacting with the filter **20,** the charged minute water particles have its radical species functioning to remove offensive odor substances from the filter **20** for regeneration thereof.

As described in the above, the air purification apparatus of the present embodiment includes the duct **10**, the filter **20,** the electrostatically atomizing device **30,** the fan **40,** and the switching device **50.** The duct **10** has the inlet **11** and the outlet **12.** The filter **20** is placed the inside of the duct **10**. The electrostatically atomizing device **30** is configured to generate a mist of charged minute water particles by means of electrostatically atomizing phenomenon and discharge the same into the duct **10**. The fan **40** is configured to send air from the inlet **11** to the outlet **12**. The switching means **50** is configured to switch one of the first mode and the second mode. The air purification apparatus sends a mist of charged minute water particles from the electrostatically atomizing device **30** to the outlet **12** not through the filter **20** in the first mode. The air purification apparatus sends a mist of charged minute water particles from the electrostatically atomizing device **30** to the filter **20** in the second mode.

Thus, the air purification apparatus of the present embodiment can purify air in the first mode, and can regenerate the filter **20** in the second mode. Further, the switching device **50** can switch one of the first mode and the second mode.

Consequently, the air purification apparatus of the present embodiment is capable of making both purification of air and regeneration of the filter **20** by use of the single electrostatically atomizing device **30.** Therefore, it is possible to downsize the air purification apparatus, and to reduce a production cost. In addition, in the first mode, a mist of charged minute water particles is discharged out from the duct **10** not through the filter **20.** Thus, it is possible to prevent occurrence of a decrease of an amount of a mist of charged minute water particles discharged out from the duct **10** caused by the filter **20**. As a result, it is possible to provide an enough amount of a mist of charged minute water particles discharged out from the duct **10.** Moreover, since the filter **20** can be regenerated in the second mode, it is possible to reduce replacement frequency of filter **20.** Thus, maintenance need not be made for a long time.

Especially, the electrostatically atomizing device **30** is configured to discharge a mist of charged minute water particles into the space formed inside of the duct **10** between the filter **20** and the outlet **12.**

The switching device **50** includes the electric motor **51** as the changing means, and the control unit **52** configured to control the electric motor **51**. The electric motor **51** is configured to change the discharge direction **M.** The control unit **52** is configured to direct the discharge direction **M** toward the outlet **12** when the first mode is selected, and to direct the discharge direction **M** toward the filter **20** when the second mode is selected.

Accordingly, it is possible to easily and successfully select one of the first mode and the second mode.

Additionally, in the second mode, the discharge electrode **320** has the second configuration temperature. Therefore, dew generated on the surface of the discharge electrode **320** is greater in the second mode than in the first mode. As a result, a generation amount of a mist of charged minute water particles is increased.

In other words, the electrostatically atomizing device **30** is configured such that an amount of a mist of charged minute water particles generated per unit time is greater in the second mode than in the first mode.

Therefore, in contrast to a situation where an amount of a mist of charged minute water particles generated per unit time is the same in the second mode as in the first mode, it is possible to shorten time necessitated for regeneration of the filter.

Beside, a large amount of ozone is generated while a large amount of a mist of charged minute water particles is generated. However, since both ozone and a mist of charged minute water particles come into contact with the filter **20,** the filter **20** prevents both ozone and a mist of charged minute water particles from being discharged out. Therefore, it is possible to assure safety of the air purification apparatus.

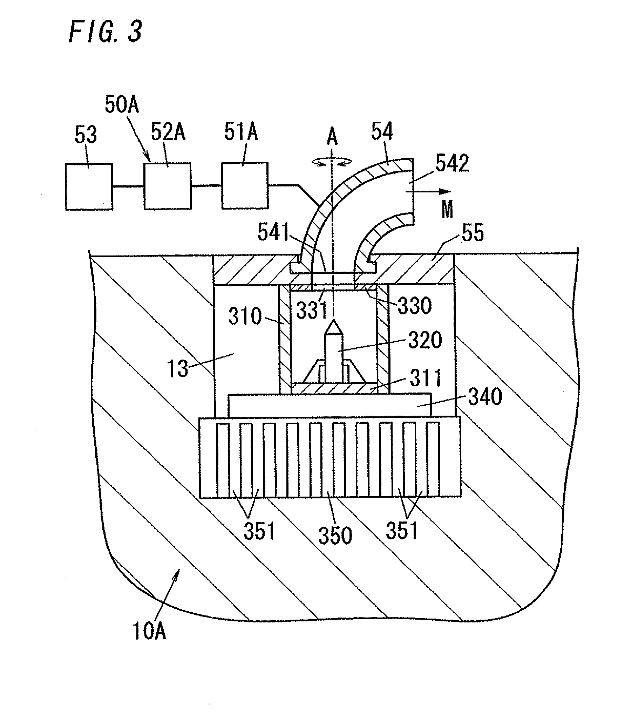

FIG. 3 shows a modification of the air purification apparatus of the present embodiment. Besides, the circuit components of the electrostatically atomizing device **30** are not shown in FIG. 3.

In the modification shown in FIG. 3, the duct **10A** is provided in its inner surface with a storing recess **13** configured to receive the electrostatically atomizing device **30.** The storing recess **13** is located between the outlet **12** and the filter **20.**

The electrostatically atomizing device **30** is accommodated within the storing recess with its spray hole **331** directed towards an opening of the storing recess **13.**

The switching device **50A** shown in Fig. 3 includes the electric motor **51A,** the control unit **52,** the manipulation unit **53,** a pipe **54,** and a bearing plate **55.**

The pipe **54** is shaped into a cylindrical shape. The pipe **54** is provided with a first opening **541** in its first axial end, and provided with a second opening **542** in its second axial end. The pipe **54** has an arc-shaped axis having a central angle of 90 degree. Therefore, the first opening **541** has its central axis perpendicular to a central axis of the second opening **542.**

The bearing plate **55** is attached to the duct **10A** to cover the storing recess **13.** The bearing plate **55** bears the pipe **54** such that the first opening **541** and the spray hole **331** communicate with each other. Additionally, the bearing plate **55** is configured to hold the pipe **54** rotatively around a rotation axis **A** (see FIG.3). The rotation axis **A** is aligned with the central axis of the first opening **541**.

The electric motor **51A** is configured to rotate the pipe **54** around the rotation axis **A.** The electric motor **51A** is a stepping motor, for example. The electric motor **51A** has a stator (not shown) fixed to the bearing plate **55.** The electric motor **51A** includes a rotor (not shown) which is mechanically coupled to the pipe **54** such that the pipe **54** rotates with a rotation of the rotor.

In the instance shown in FIG. 3, a mist of charged minute water particles is discharged into the duct **10** from the electrostatically atomizing device **30** through the pipe **54.** A direction toward which the second opening **542** is directed is defined as the discharge direction **M.** The discharge direction **M** is perpendicular to the rotation axis **A,** because of that the discharge direction **M** is aligned with the central axis of the second opening **542.**

Consequently, the discharge direction **M** is changed with a rotation of the pipe **54.** In the instance shown in FIG. 3, the electric motor **51A,** the pipe **54,** and the bearing plate **55** constitute the changing means configured to change the discharge direction **M.**

In addition, as described in the above, the storage recess **13** is located between the outlet **12** and the filter **20.** Therefore, the electrostatically atomizing device **30** discharges a mist of charged minute water particles into the space formed inside of the duct **10** between the filter **20** and the outlet **12.**

The control unit **52A** is configured to control the electric motor **51A,** the fan **40,** and the control circuit **390** in accordance with the operation mode of the air purification apparatus. Besides, the control unit **52A** controls the fan **40** and the control circuit **390** in a similar manner as seen in the control unit **52,** and no explanation thereof is deemed necessary.

When the first mode is selected, the control unit **52A** rotates the pipe **54** to direct the discharge direction **M** toward the outlet **12.** Thus, in the first mode, a mist of charged minute water particles is discharged toward the outlet **12** from the electrostatically atomizing device **30.** Further, since the fan **40** sends air towards the downstream side, a mist of charged minute water particles is moved towards the outlet **12** as being carried on an air flow.

When the second mode is selected, the control unit **52A** rotates the pipe **54** to direct the discharge direction **M** towards the filter **20.** Thus, in the second mode, a mist of charged minute water particles is discharged from the electrostatic atomizing device **30** towards the filter **20.** Further, since the fan **40** sends air towards the upstream side, a mist of charged minute water particles is moved towards the filter **20** as being carried on an air flow.

Accordingly, the modification of the air purification apparatus shown in FIG. 3 also can provide the same effect as the instance shown in FIG. 1.

Besides, the air purification apparatus of the present embodiment and the following second and third embodiments is defined as an apparatus having at least an air purification function, and therefore may be an air conditioner of cooling and/or heating air, a humidifier, a dehumidifier, a humidification and air purification apparatus, a vacuum cleanser, or a fan heater.

In addition, the filter **20** used in the present embodiment and the following second and third embodiments is not limited to a deodorization filter. The filter **20** may be a dust collection filter (e.g., HEPA filter) configured to collect particles floating in the air, or a sterilization filter designed to remove bacteria and viruses. Alternatively, the filter **20** may have both a function of deodorization and a function of removing dusts, bacteria, and viruses. A filter having a desired function can be adopted as the filter **20.** Moreover, a mist of charged minute water particles can regenerate a sterilization filter, because of that a mist of charged minute water particles inactivates bacteria and viruses.

### (second embodiment)

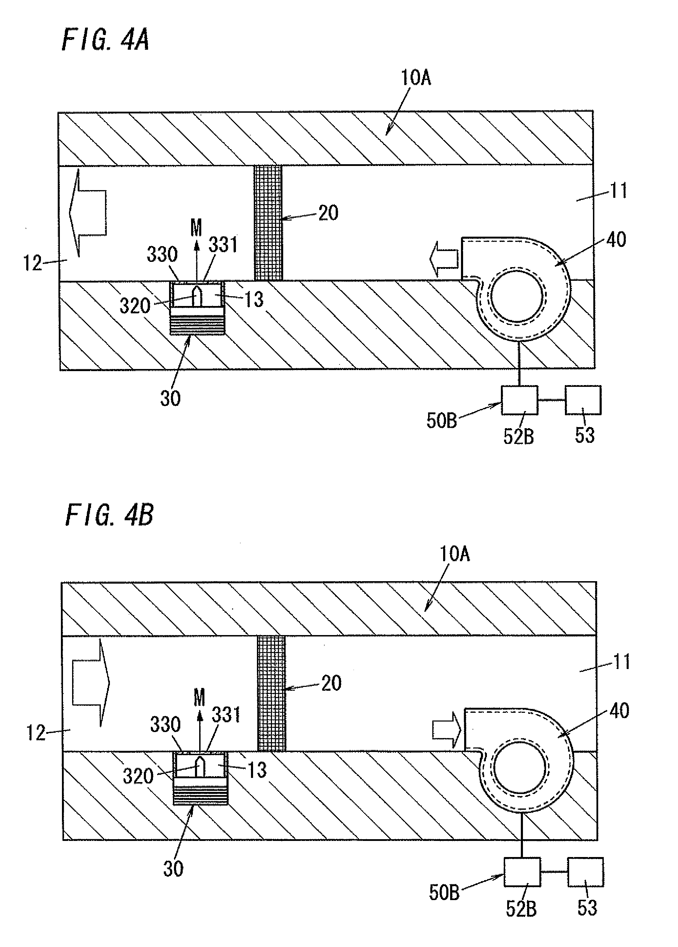

As shown in FIG. 4, the air purification apparatus of the present embodiment is mainly different from that of the first embodiment in the switching device **50B.** Besides, with regard to components common to the present embodiment and the first embodiment, no detailed explanations are deemed necessary.

In the air purification apparatus of the present embodiment, like the instance shown in FIG. 3, the electrostatically atomizing device **30** is accommodated within the storage recess **13** of the duct **10A**.

The electrostatically atomizing device **30** is accommodated within the storing recess with its spray hole **331** directed towards the opening of the storing recess **13.** Therefore, the electrostatically atomizing device **30** discharges a mist of charged minute water particles to the space formed inside of the duct **10** between the filter **20** and the outlet **12.**

The switching device **50B** includes the control unit **52B** and the manipulation unit **53.**

The control unit **52B** is configured to control the fan **40** and the control circuit **390** in conformity with the operation mode of the air purification apparatus. The control unit **52B** selects one of the first mode and the second mode in response to manual operation of the manipulation unit **53.** Besides, the control unit **528** controls the fan **40** and the control circuit **390** in a similar manner as seen in the control unit **52** of the first embodiment, and no explanation thereof is deemed necessary.

When the first mode is selected, the control unit **52B** controls the fan **40** to operate in the air supply mode (see FIG. 4A). Therefore, in the first mode, the fan **40** sends air towards the downstream side. Thus, a mist of charged minute water particles discharged between the outlet **12** and the filter **20** is moved towards the outlet **12** as being carried on an air flow. Accordingly, in the first mode, a mist of charged minute water particles is discharged out from the outlet **12** without contacting with the filter **20.**

When the second mode is selected, the control unit **52B** controls the fan **40** to operate in the exhaust mode (see FIG. 4B). Therefore, in the second mode, the fan **40** sends air towards the upstream side. Thus, a mist of charged minute water particles discharged between the outlet **12** and the filter **20** is moved towards the filter **20** as being carried on an air flow. Accordingly, in the second mode, a mist of charged minute water particles contacts with the filter **20.**

As seen from the above, the switching device **50B** of the present embodiment includes the control circuit **52B** defined as a control means configured to control the fan **40.** The control circuit **52B** controls the fan **40** to operate in the air supply mode when the first mode is selected, and controls the fan **40** to operate in the exhaust mode when the second mode is selected.

As described in the above, in the air purification apparatus of the present embodiment, the electrostatically atomizing device **30** is configured to discharge a mist of charged minute water particles into the space formed inside of the duct **10** between the filter **20** and the outlet **12.** The fan **40** is configured to operate in the air supply mode of sending air from the inlet **11** to the outlet **12,** and the exhaust mode of sending air from the outlet **12** to the inlet **11.** The switching device **50B** includes the control circuit **52B** configured to control the fan **40.** The control circuit **52B** is configured to control the fan **40** to operate in the air supply mode when the first mode is selected, and to control the fan **40** to operate in the exhaust mode when the second mode is selected.

Consequently, the air purification apparatus of the present embodiment also produces the same effect as the air purification apparatus of the first embodiment. Especially, in the present embodiment, the first mode and the second mode are switched by only switching the working mode between the air supply mode and the exhaust mode. Therefore, it is possible to easily and successfully select one of the first mode and the second mode. In addition, it is possible to reduce the production cost, because of that the electric motor **51** is unnecessary.

### (third embodiment)

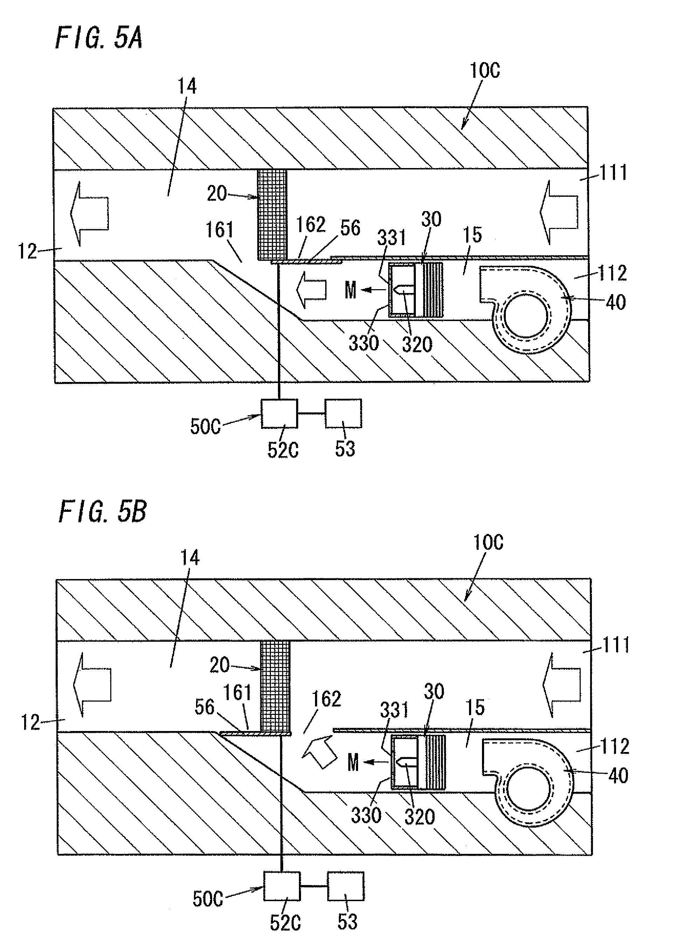

As shown in FIG. 5, the air purification apparatus of the present embodiment is mainly different from the first embodiment in the configurations of the duct **10C** and the switching device **50C.** Besides, with regard to components common to the present embodiment and the first embodiment, no detailed explanations are deemed necessary.

The duct **10C** is provided with a first inlet **111** and a second inlet **112.** The duct **10C** has a main channel **14** connecting the first inlet **111** to the outlet **12.** The duct **10C** has an auxiliary channel **15** connecting the second inlet **112** to the outlet **12.** The duct **10C** includes a first confluent opening **161** and a second confluent opening **162** respectively connecting the main channel **14** to the auxiliary channel **15.**

The first confluent opening **161** connects the auxiliary channel **15** to the main channel **14** between the outlet **12** and the filter **20** (that is, downstream side). The second confluent opening **162** connects the auxiliary channel **15** to the main channel **14** between the first inlet **111** and the filter **20** (that is, upstream side).

In the present embodiment, the electrostatically atomizing device **30** is located inside of the auxiliary channel **15** while the spray hole **331** is directed toward the outlet **12.** In the present embodiment, the electrostatically atomizing device **30** is located in a center part of the auxiliary channel **15** not to occlude the auxiliary channel **15.**

In addition, the fan **40** is installed in the duct **10C** to send air from the second inlet **112** (upstream side) to the electrostatically atomizing device **30** (downstream side).

As seen from the above, in the air purification apparatus of the present embodiment, the duct **10C** has two air passages (that is, a first air passage and a second air passage) between the outlet **12** and the electrostatically atomizing device **30.**

The first air passage is defined by the auxiliary channel **15,** the first confluent opening **161,** and the main channel **14.** The first air passage is devoid of the filter **20** between the outlet **12** and the electrostatically atomizing device **30** (see FIG. 5A).

The second air passage is defined by the auxiliary channel **15,** the second confluent opening **162**, and the main channel **14.** The second air passage is provided with the filter **20** between the outlet **12** and the electrostatically atomizing device **30** (see FIG. 5B).

The switching device **10C** includes a shutter **56,** the control unit **52C,** and the manipulation unit **53.**

The shutter **56** is configured to move (slide) between a first position in which the shutter **56** close only the first confluent opening **161** and a second position in which the shutter **56** close only the second confluent opening **162**. In other words, the shutter **56** is defined as a switching valve configured to close the first air passage and the second air passage selectively. As apparent from the above, the air purification apparatus of the present embodiment is provided with a confluence selecting structure for selecting a confluence of the main channel **14** with the auxiliary channel **15** from which one of the points downstream and upstream of the filter **20.**

The control unit **52C** is configured to control the shutter **56** and the control circuit **390** in accordance with the operation mode of the air purification apparatus. The control unit **52C** selects one of the first mode and the second mode in response to manual operation of the manipulation unit **53.** Besides, the control unit **52C** controls the control circuit **390** in the same manner as the control unit **52** of the first embodiment, and therefore no explanation thereof is deemed necessary. The fan **40** operates in the air supply mode irrespective of selection of the first mode and the second mode.

The control unit **52C** places the shutter **56** in the second position when the first mode is selected (see FIG. 5A). Therefore, in the first mode, the second air passage is kept closed, and the first air passage is kept opened. Thus, a mist of charged minute water particles discharged from the electrostatically atomizing device **30** passes the first air passage. As a result, in the first mode, a mist of charged minute water particles is discharged into the external space via the outlet **12** not through the filter **20.**

The control unit **52C** places the shutter **56** in the first position when the second mode is selected (see FIG. 5B). Therefore, in the second mode, the first air passage is kept closed, and the second air passage is kept opened. Thus, a mist of charged minute water particles discharged from the electrostatically atomizing device **30** passes the second air passage. As a result, in the second mode, a mist of charged minute water particles is allowed to contact with the filter **20.**

Besides, irrespective of selection of the first mode and the second mode, air coming into the duct **10C** via the first inlet **111** is discharged out from the outlet **12** through the filter **20.**

As described in the above, in the air purification apparatus of the present embodiment, the duct **10C** includes the first air passage and the second air passage. The first air passage is not provided with the filter **20** between the outlet **12** and the electrostatically atomizing device **30,** and the second air passage is provided with the filter **20** between the outlet **12** and the electrostatically atomizing device **30.** The switching device **50C** includes the shutter **56** configured to shut the first air passage and the second air passage selectively, and the control unit **52C** configured to control the shutter **56.** The control unit **52C** is configured to control the shutter **56** to block the second air passage when the first mode is selected, and to control the shutter **56** to block the first air passage when the second mode is selected.

Accordingly, the air purification apparatus of the present embodiment also gives the same effect as the air purification apparatus of the first embodiment. Especially, in the present embodiment, the shutter **56** is moved to switch one of the first mode and the second mode. Therefore, it is possible to easily and successfully select one of the first mode and the second mode,

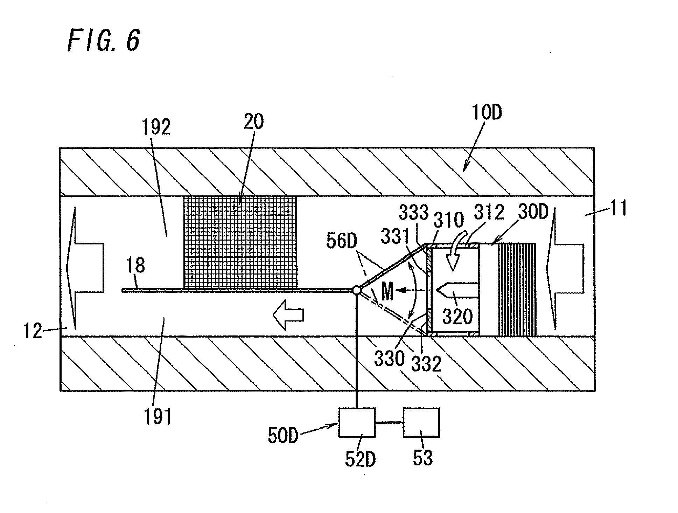

FIG. 6 shows a modification of the air purification apparatus of the present embodiment.

In the instance shown in FIG. 6, the duct **10D** includes the inlet **11** and the outlet **12.**

The electrostatically atomizing apparatus **30D** is located close to the inlet **11** within the duct **10D.** The electrostatically atomizing device **30** has its spray hole **331** directed toward the outlet **12**. The electrostatically atomizing device **30** shown in FIG. 6 is provided with an air hole **312** in its side wall. Partial air coming into the duct **10D** via the inlet **11** comes into the housing **310** through the air hole **312**.

The duct **10D** shown in FIG.6 is provided with a partition wall **18** between the outlet **12** and the electrostatically atomizing device **30.** The partition wall **18** divides the inner space of the duct **10D** in two portions in order to form a first channel **191** and a second channel **192.** With regard to the instance shown in FIG. 6, the filter **20** is placed inside of the second channel **192.**

As apparent from the above, in the air purification apparatus shown in FIG. 6, the duct **10D** has two air passage (i.e., first air passage and second air passage) between the outlet **12** and the electrostatically atomizing device **30.**

The first air passage is defined by the first channel **191** in association with a space between the electrostatically atomizing device **30** and the partition wall **18.** The first air passage is not provided with the filter **20** between the outlet **12** and the electrostatically atomizing device **30.**

The second air passage is defined by the second channel **192** in association with the space between the electrostatically atomizing device **30** and the partition wall **18.** The second air passage is provided with the filter **20** between the outlet **12** and the electrostatically atomizing device **30.**

The switching device **50D** shown in FIG. 6 includes the shutter **56D,** the control unit **52D,** and the manipulation unit **53.**

The shutter **56D** is attached to an end of the partition wall **18** adjacent to the inlet **11** so as to move (rotate) between a first position and a second position.

The first position is defined as a position (position of the shutter **56D** illustrated with broken lines in FIG. 6) in which the shutter **56D** blocks a clearance between the partition wall **18** and a first end (lower end, in FIG. 6) **332** of the opposed electrode **330** in order to prevent a mist of charged minute water particles from coming into the first channel **191**.

The second position is defined as a position (position of the shutter **56D** illustrated with solid lines in FIG. 6) in which the shutter **56D** blocks a clearance between the partition wall **18** and a second end (upper end, in FIG. 6) **333** of the opposed electrode **330** in order to prevent a mist of charged minute water particles from coming into the second channel **192.**

In brief, the shutter **56D** is defined as a switching valve configured to close one of the first air passage and the second air passage. As apparent from the above, the air purification apparatus of the present embodiment is provided with a confluence switching structure for selecting one of the first channel **191** and the second channel **192.**

The control unit **52D** is configured to control the shutter **56D** and the control circuit **390** in accordance with the operation mode of the air purification apparatus. The control unit **52D** selects one of the first mode and the second mode in response to manual operation of the manipulation unit **53.** Besides, the control unit **52D** controls the control circuit **390** in the same manner as the control unit **52** of the first embodiment, and therefore no explanation thereof is deemed necessary.

The control unit **52D** places the shutter **56D** in the second position when the first mode is selected. Therefore, in the first mode, the second air passage is kept closed, and the first air passage is kept opened. Thus, a mist of charged minute water particles discharged from the electrostatically atomizing device **30** passes the first air passage. As a result, in the first mode, a mist of charged minute water particles is discharged into the external space via the outlet **12** without said particles contacting with the filter **20.**

The control unit **52D** places the shutter **56D** in the first position when the second mode is selected. Therefore, in the second mode, the first air passage is kept closed, and the second air passage is kept opened. Thus, a mist of charged minute water particles discharged from the electrostatically atomizing device **30** passes the second air passage. As a result, in the second mode, a mist of charged minute water particles is allowed to contact with the filter **20.**

Besides, in both the first mode and the second mode, air coming into the duct **10D** via the inlet **11** is discharged out from the outlet **12** through the filter **20.**

The modification of the air purification apparatus shown in FIG. 6 also provides the same effect as the air purification apparatus shown in FIG. 5.

## Claims

1. An air purification apparatus comprising:
a duct having an inlet and an outlet;
a filter designed to purify air and placed inside of said duct;
an electrostatically atomizing device configured to generate a mist of charged minute water particles by means of electrostatically atomizing phenomenon and discharge the same into said duct; and
a fan configured to send air from said inlet to said outlet,
wherein said air purification apparatus comprises a switching means configured to switch one of a first mode and a second mode, and
said air purification apparatus being configured to send a mist of charged minute water particles from said electrostatically atomizing device to said outlet not through said filter in said first mode, and to send a mist of charged minute water particles from said electrostatically atomizing device to said filter in said second mode.

2. An air purification apparatus as set forth in claim 1, wherein
said electrostatically atomizing device is configured to discharge a mist of charged minute water particles into a space formed inside of said duct between said filter and said outlet,
said switching means including a changing means configured to change a discharge direction where said electrostatically atomizing device discharges a mist of charged minute water particles, and a controlling means configured to control said changing means, and
said controlling means being configured to direct said discharge direction toward said outlet in said first mode, and to direct said discharge direction toward said filter in said second mode.

3. An air purification apparatus as set forth in claim 1, wherein
said electrostatically atomizing device is configured to discharge a mist of charged minute water particles into a space formed inside of said duct between said filter and said outlet,
said fan being configured to operate in an air supply mode of sending air from said inlet to said outlet, and an exhaust mode of sending air from said outlet to said inlet,
said switching means including a controlling means configured to control said fan, and said controlling means being configured to control said fan to operate in said air supply mode when said first mode is selected, and to control said fan to operate in said exhaust mode when said second mode is selected.

4. An air purification apparatus as set forth in claim 1, wherein
said electrostatically atomizing device is placed inside of said duct,
said duct including, between said outlet and said electrostatically atomizing device, a first air passage devoid of said filter and a second air passage provided with said filter,
said switching means including a shutter configured to block said first air passage and said second air passage selectively, and a controlling means configured to control said shutter, and
said controlling means being configured to control said shutter to block said second air passage when said first mode is selected, and to control said shutter to block said first air passage when said second mode is selected.

5. An air purification apparatus as set forth in claim 1, wherein
said electrostatically atomizing device is configured such that an amount of a mist of charged minute water particles generated per unit time is greater in said second mode than in said first mode.

## Amended claims

### Amended claims under Art. 19.1 PCT

**1.** An air purification apparatus comprising:
a duct having an inlet and an outlet;
a filter designed to purify air and placed inside of said duct;
an electrostatically atomizing device configured to generate a mist of charged minute water particles by means of electrostatically atomizing phenomenon and discharge the same into said duct; and
a fan configured to send air from said inlet to said outlet,
wherein said air purification apparatus comprises a switching means configured to switch one of a first mode of purifying air and a second mode of regenerating said filter, and
said air purification apparatus being configured to discharge a mist of charged minute water particles generated by said electrostatically atomizing device into an external space via said outlet not through said filter in said first mode, and to make the mist of charged minute water particles generated by said electrostatically atomizing device into contact with said filter in said second mode.

**2.** An air purification apparatus as set forth in claim 1, wherein
said electrostatically atomizing device is configured to discharge a mist of charged minute water particles into a space formed inside of said duct between said filter and said outlet,
said switching means including a changing means configured to change a discharge direction where said electrostatically atomizing device discharges a mist of charged minute water particles, and a controlling means configured to control said changing means, and
said controlling means being configured to direct said discharge direction toward said outlet in said first mode, and to direct said discharge direction toward said filter in said second mode.

**3.** An air purification apparatus as set forth in claim 1, wherein
said electrostatically atomizing device is configured to discharge a mist of charged minute water particles into a space formed inside of said duct between said filter and said outlet,
said fan being configured to operate in an air supply mode of sending air from said inlet to said outlet, and an exhaust mode of sending air from said outlet to said inlet,
said switching means including a controlling means configured to control said fan, and said controlling means being configured to control said fan to operate in said air supply mode when said first mode is selected, and to control said fan to operate in said exhaust mode when said second mode is selected.

**4.** An air purification apparatus as set forth in claim 1, wherein
said electrostatically atomizing device is placed inside of said duct,
said duct including, between said outlet and said electrostatically atomizing device, a first air passage devoid of said filter and a second air passage provided with said filter,
said switching means including a shutter configured to block said first air passage and said second air passage selectively, and a controlling means configured to control said shutter, and
said controlling means being configured to control said shutter to block said second air passage when said first mode is selected, and to control said shutter to block said first air passage when said second mode is selected.

**5.** An air purification apparatus as set forth in claim 1, wherein
said electrostatically atomizing device is configured such that an amount of a mist of charged minute water particles generated per unit time is greater in said second mode than in said first mode.
